# FASCICULE DE BREVET EUROPEEN

(11) **EP 4 408 317 B1**
(45) Date de publication et mention de la délivrance du brevet: **11.02.2026**
(21) Numéro de dépôt: 22797691.7
(22) Date de dépôt: 27.09.2022
(51) Int. Cl.: A61B 17/66

(54) **DISPOSITIF DE POSITIONNEMENT DES OS MAXILLAIRES POUR LA CHIRURGIE ORTHOGNATHIQUE**
VORRICHTUNG ZUR POSITIONIERUNG DER KIEFERKNOCHEN FÜR DIE ORTHOGNATISCHE CHIRURGIE
DEVICE FOR POSITIONING THE MAXILLARY BONES FOR ORTHOGNATHIC SURGERY

(30) Priorité: 29.09.2021 FR 2110258
(43) Date de publication de la demande: 07.08.2024
(73) Titulaire: Ennoia, 25000 Besançon (FR)
(72) Inventeur: BILLOTTET, Benjamin, 25480 Miserey Salines (FR)
(74) Mandataire: Radzimski, Eric
(86) Numéro de dépôt international: PCT/EP2022/076771
(87) Numéro de publication internationale: WO 2023/052328

(56) Documents cités:
- US-A1- 2003 097 137
- US-A1- 2015 044 624

## Description

### Domaine de l'invention

La présente invention concerne le domaine des équipements médicaux aptes à être utilisés par un chirurgien au cours d'une opération chirurgicale.

En particulier, l'invention concerne un dispositif médical spécifiquement adapté à la chirurgie maxillo-faciale, et plus précisément à la chirurgie orthognathique.

### Art antérieur

La chirurgie orthognathique est une activité spécifique de la chirurgie maxillo-faciale, qui vise à rendre les os du maxillaire et de la mâchoire droits et alignés, en intervenant sur les os des maxillaires. Cette chirurgie a notamment pour objectif de rétablir un positionnement adapté des dents du patient, afin d'obtenir un bon mordu et une bonne occlusion dentaire.

Une telle opération chirurgicale consiste en une section osseuse, ou ostéotomie, suivie d'un repositionnement des bases osseuses qui sont fixées dans leur nouvelle position par des plaques d'ostéosynthèse maintenues par des vis. Cette section osseuse peut concerner le maxillaire, le mandibulaire ou le menton.

La chirurgie du maxillaire, appelée « ostéotomie de Lefort 1 », consiste ainsi à découper les os maxillaires, afin de séparer la portion inférieure des os maxillaires, qui porte l'arcade dentaire supérieure, des parties supérieures des os maxillaires, qui sont reliés aux autres os du crâne. Cette séparation a pour objectif de repositionner la portion inférieure par rapport à la mâchoire inférieure. La présente invention concerne un équipement de positionnement spécifiquement adapté à une telle chirurgie du maxillaire, ou « ostéotomie de Lefort 1 ».

La réalisation d'une « ostéotomie de Lefort 1 » est précédée d'une analyse, par le chirurgien, de la position optimale de la portion inférieure du maxillaire, permettant notamment de corriger les dysmorphoses et d'obtenir une occlusion dentaire optimale. Cette analyse se fait notamment sur la base de radiographies spéciales du crâne et de la face et prend en compte de nombreux facteurs cliniques, dentaires, morphologiques et psychologiques.

Quand la position optimale de la portion inférieure du maxillaire est déterminée, il est nécessaire de préparer des moyens de positionnement du maxillaire, pouvant être mis en œuvre après l'ostéotomie pour maintenir et fixer ce maxillaire dans la position optimale. Plusieurs dispositifs de maintien existent pour assurer un tel positionnement.

On connaît ainsi des gouttières chirurgicales, réalisées souvent par des prothésistes dentaires et présentant des formes complémentaires de la dentition du patient. De telles gouttières sont basées sur des systèmes mécaniques, appelés articulateurs dentaires, permettant des mouvements relatifs contrôlés de l'arcade dentaire supérieure par rapport à l'arcade dentaire inférieure. De telles gouttières chirurgicales peuvent par exemple être fabriquées par une impression en trois dimensions basée sur une modélisation numérique des arcades dentaires et une simulation du geste chirurgical à réaliser.

Il est également connu d'assurer le positionnement du maxillaire supérieur à l'aide de guides chirurgicaux adaptés. De tels guides chirurgicaux sont souvent réalisés sur mesure, à partir d'une imagerie médicale en trois dimensions du patient et d'une simulation du geste chirurgical à réaliser. Il existe également différents types de guides chirurgicaux spécifiques, adaptés chacun à un type particulier de déplacement de la partie inférieure d'un maxillaire par rapport à la partie supérieure de ce maxillaire. La demande de brevet US20120277749 décrit ainsi un ensemble de tels guides chirurgicaux spécifiques, permettant de repositionner la mâchoire supérieure.

US 2003/097137 A1 décrit un dispositif pour l'expansion et/ou la rétraction de la mâchoire maxillaire, configuré pour être monté dans la bouche et reliant les moitiés droite et gauche du crâne.

### Inconvénients de l'art antérieur

Les dispositifs de positionnement qui sont réalisés sur mesure pour chaque patient sont onéreux, et leur préparation s'avère contraignante. En effet, celle-ci impose au patient plusieurs étapes d'imagerie médicale ou de moulage dentaire au cours de la face préopératoire.

Par ailleurs, ces dispositifs de positionnement réalisés sur mesure imposent au chirurgien une anticipation parfaite du geste chirurgical à réaliser. Il doit ainsi anticiper les déplacements exacts des pièces osseuses dans l'espace. En effet, ce geste chirurgical et la position finale du maxillaire ne peuvent pas être modifiés ou adaptés facilement, après la fabrication du dispositif de positionnement. Ces dispositifs de positionnement réalisés sur mesure ne laissent donc que peu de liberté de modification possible par le chirurgien. Enfin, de tels dispositifs de positionnement réalisés sur mesure sont particulièrement onéreux.

Les guides chirurgicaux sont également définis précisément avant l'opération, et souvent fabriqués sur mesure. De tels guides chirurgicaux ne permettent que des déplacements des bases osseuses dans une direction, ou dans des directions qui ont été prédéfinies lors de la phase préopératoire. Un guide chirurgical spécifique est choisi et adapté pour une opération chirurgicale. Il ne permet pas au chirurgien, lors de l'opération, de réaliser un déplacement des bases osseuses différent du geste chirurgical prévu.

### Objectifs de l'invention

La présente invention a pour objectif de pallier ces inconvénients de l'art antérieur.

En particulier, la présente invention a pour objectif de fournir un dispositif de positionnement du maxillaire, dans le cadre d'une chirurgie orthognathique du maxillaire, qui permette au chirurgien d'assurer un repositionnement facile de l'arcade dentaire supérieure par rapport à l'arcade dentaire inférieure.

Un autre objectif de l'invention est de fournir un tel dispositif de positionnement dont le coût est inférieur à celui des solutions de l'art antérieur.

Un autre objectif de l'invention est de fournir un tel dispositif de positionnement qui permette de limiter la durée et le coût des étapes de préparation de l'opération.

Un objectif particulier de l'invention est de fournir un tel dispositif de positionnement qui soit standard, ajustable, et puisse s'adapter à l'ensemble des patients.

Enfin, un autre objectif particulier de l'invention est de fournir un tel dispositif de positionnement qui permette au chirurgien de réaliser un ajustement précis de la position de la portion inférieure des os maxillaires dans le cadre d'une ostéotomie de Lefort et, si cela s'avère nécessaire, de modifier ou d'ajuster cette position jusqu'à la fixation de la portion inférieure des os maxillaires par rapport aux portions supérieures des os maxillaires.

Un autre objectif est de pouvoir, lors de l'utilisation, obtenir un retour sensoriel et plus particulièrement sonore afin de fournir des indications relatives aux déplacements effectués par translation.

### Exposé de l'invention

Ces objectifs, ainsi que d'autres qui apparaîtront plus clairement par la suite, sont atteints à l'aide d'un dispositif de positionnement des os maxillaires dans le cadre d'une opération chirurgicale d'ostéotomie des os maxillaires d'un patient.

Selon l'invention, ce dispositif de positionnement des os maxillaires est caractérisé en ce qu'il comprend :
- un élément d'ancrage central, apte à être fixé aux os maxillaires du patient, entre son nez et son arcade dentaire,
- deux éléments d'ancrage latéraux, aptes à être fixés aux os maxillaires du patient, chacun de l'un des côtés du nez du patient,
- une structure de liaison connectée aux éléments d'ancrage latéraux et central, la structure de liaison étant articulée pour former trois liaisons pivot-glissant entre l'élément d'ancrage central et chacun des éléments d'ancrage latéraux :
   - une première liaison pivot-glissant, orientée selon un premier axe,
   - une deuxième liaison pivot-glissant, orientée selon un deuxième axe sensiblement orthogonal au premier axe, et
   - une troisième liaison pivot-glissant, orientée selon un troisième axe sensiblement orthogonal au deuxième axe.

Ces liaisons pivot permettent d'ajuster finement la position de la portion inférieure des os maxillaires par rapport aux portions supérieures des os maxillaires. Ainsi, le chirurgien peut réaliser des déplacements de cette portion inférieure des os maxillaires dans différents plans de l'espace par rapport à la tête du patient. Le chirurgien possède une grande liberté pour effectuer ces déplacements de la portion inférieure des os maxillaires, y compris, si nécessaire, en modifiant au cours de l'opération le protocole prévu préalablement.

Le fait qu'un axe soit « sensiblement orthogonal » à un autre axe signifie, dans la présente description, que l'un de ces axes est orienté selon un angle inférieur à 10 degrés avec une direction orthogonale à l'autre axe.

De préférence, la structure de liaison comprend plusieurs pièces mobiles les unes par rapport aux autres, parmi lesquelles :
- une première pièce connectée à l'élément d'ancrage central par la première liaison pivot-glissant, et
- une deuxième pièce connectée à la première pièce par une des deuxième liaison pivot-glissant, et connectée à un premier des éléments d'ancrage latéraux, par une des troisième liaison pivot-glissant.

Selon un mode de réalisation avantageux, la structure de liaison comprend :
- une troisième pièce connectée à la première pièce par une liaison glissière orientée selon un axe parallèle au premier axe, et
- une quatrième pièce connectée à la troisième pièce par une des deuxième liaison pivot-glissant, et connectée au second des éléments d'ancrage latéraux, par une des troisième liaison pivot-glissant.

De préférence, au moins une des liaisons pivot-glissant est constituée par une ouverture ménagée dans une pièce, dans laquelle est introduite une tige cylindrique, cette tige présentant une pluralité de rainures périphériques réparties sur au moins une partie de sa longueur.

Ces rainures périphériques permettent au chirurgien d'avoir des repères pour les déplacements des tiges dans l'ouverture.

Avantageusement, cette liaison pivot-glissant est équipée d'un curseur placé dans l'ouverture, ce curseur étant apte à coopérer avec une rainure de la tige pour maintenir la position en translation de la tige dans l'ouverture, tant qu'une force prédéterminée n'est pas appliquée.

De préférence, ce curseur est conformé pour générer un son lors de son passage d'une des rainures à une autre des rainures de la tige.

Ainsi, les mouvements de translation de chaque liaison pivot-glissant peuvent être finement contrôlés par le chirurgien, les rainures lui offrant un retour sensitif et sonore pour chaque déplacement d'un pas prédéterminé. Ce pas, qui correspond à l'écart entre deux rainures successives, peut par exemple être de 1 mm.

Préférentiellement, le dispositif de positionnement est équipé de moyens de blocage réversible d'au moins une des liaisons pivot-glissant.

Ainsi, lorsque le chirurgien obtient la position souhaitée de la portion inférieure des os maxillaires par rapport aux portions supérieures des os maxillaires du patient, il peut bloquer la position des éléments constituant les secondes liaisons pivot-glissant afin de maintenir cette position.

Selon un mode de réalisation avantageux, au moins un des moyens de blocage réversible comprend une came excentrique associée à un levier.

### Liste des figures

L'invention sera mieux comprise à la lecture de la description suivante de modes de réalisation préférentiels, donnée à titre de simple exemple figuratif et non limitatif, et accompagnée des figures parmi lesquelles :
- [Fig.1] la [Fig.1] est une représentation schématique d'un crâne humain découpé pour une opération chirurgicale d'« ostéotomie de Lefort 1 ».
- [Fig.2] la [Fig.2] est une représentation en perspective d'un dispositif de positionnement selon un mode de réalisation de l'invention.
- [Fig.3] la [Fig.3] est une représentation en perspective des éléments d'ancrage du dispositif de positionnement de la [Fig.2], fixés aux os maxillaires d'un patient.
- [Fig.4] la [Fig.4] est une représentation en perspective de la structure de liaison du dispositif de positionnement de la [Fig.2].
- [Fig.5] la [Fig.5] est une vue en perspective du dispositif de positionnement de la [Fig.2] fixé sur les os maxillaires d'un patient.

### Description détaillée de modes de réalisation de l'invention

### 1. Dispositif de positionnement

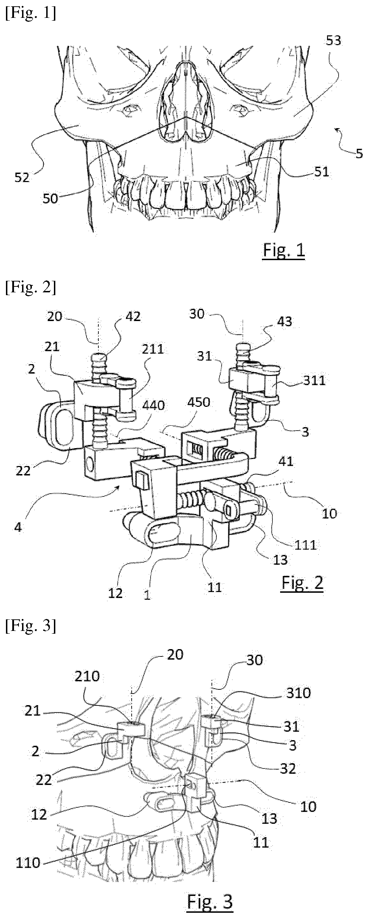

La [Fig.2] représente en perspective un dispositif de positionnement selon un mode de réalisation de l'invention. Ce dispositif de positionnement est composé de plusieurs éléments. Il comporte ainsi un élément d'ancrage central 1, deux éléments d'ancrage latéraux, respectivement 2 et 3, et une structure de liaison 4 qui est connectée à chacun des éléments d'ancrage central et latéraux et qui les relie entre eux.

### a. Plans anatomiques

Le dispositif de positionnement de la [Fig.2] est destiné à être placé sur les os maxillaires du patient de façon symétrique par rapport au plan sagittal, ou plan médian, qui est défini comme le plan anatomique qui divise le corps en parties droite et gauche égales. Conformément aux conventions usuelles en anatomie, les termes « droite » et « gauche » désignent, dans la présente description, les objets ou parties d'objet situés respectivement à droite ou à gauche de ce plan sagittal, du point de vue du patient. Ce plan sagittal est perpendiculaire au plan frontal, qui est le plan anatomique qui sépare le corps en une partie antérieure ou ventrale et une partie postérieure ou dorsale.

### b. Os maxillaires

Le dispositif de positionnement de la [Fig.2] est spécifiquement adapté à la réalisation d'une opération chirurgicale orthognathique du maxillaire, appelée « ostéotomie de Lefort 1 ». Lors d'une telle opération chirurgicale, le chirurgien découpe chacun des os maxillaires droit et gauche, afin de séparer les portions inférieures de ces os maxillaires, qui portent l'arcade dentaire supérieure, des parties supérieures des os maxillaires, qui sont reliées aux autres os du crâne.

La [Fig.1] représente, de façon schématique, le crâne 5 d'un patient au cours d'une telle opération chirurgicale orthognathique du maxillaire. Comme le montre cette [Fig.1], après la découpe 50 effectuée par le chirurgien, les os maxillaires du patient sont décomposés en :
- une portion supérieure droite 52, placée du côté droit du plan sagittal, à côté du nez du patient, et reliée aux autres os du crâne ;
- une portion supérieure gauche 53, placée du côté gauche du plan sagittal, à côté du nez du patient, et reliée aux autres os du crâne ;
- les portions inférieures droite et gauche, qui sont soudées l'une à l'autre et portent l'arcade dentaire supérieure. L'ensemble formé par ces portions inférieures droite et gauche des os maxillaires est appelé par la suite « portion inférieure 51 » des os maxillaires.

### 2. Elément d'ancrage central

Les éléments d'ancrage du dispositif de positionnement sont destinés à être fixés aux différentes portions des os maxillaires, lors d'une opération chirurgicale orthognathique du maxillaire, comme le représente la [Fig.3].

Parmi ces éléments d'ancrage, l'élément d'ancrage central 1 présente une forme lui permettant d'être fixé par le chirurgien sur la portion inférieure 51 des os maxillaires d'un patient, entre le nez et l'arcade dentaire de celui-ci. Cet élément d'ancrage 1 comporte un élément de liaison central 11 qui est destiné à faire partie d'une liaison pivot-glissant, d'axe 10, entre l'élément d'ancrage central 1 et la structure de liaison 4, et deux pattes de fixation 12 et 13, respectivement situées à droite et à gauche de l'élément de liaison central 11.

### a. Pattes de fixation

La patte de fixation droite 12 est destinée à permettre la fixation de l'élément d'ancrage central 1 à la partie droite de la portion inférieure 51 des os maxillaires du patient, et la patte de fixation gauche 13 est destinée à permettre la fixation de l'élément d'ancrage central 1 à la partie gauche de la portion inférieure 51 des os maxillaires du patient.

Chacune de ces pattes de fixation 12 et 13 présente une surface apte à être mise en contact avec l'os du patient, et un trou de passage de vis. Chacun de ces trous permet le passage d'une vis de fixation qui peut être vissée par le chirurgien dans l'os maxillaire du patient, pour fixer l'élément d'ancrage central 1.

### b. Elément de liaison central

L'élément de liaison central 11 de l'élément d'ancrage central 1 présente une ouverture 110 dans laquelle est introduite une tige cylindrique centrale 41 de la structure de liaison 4, afin que cet élément de liaison central 11 et la tige cylindrique centrale 41 forment ensemble une liaison pivot-glissant, d'axe 10, entre l'élément d'ancrage central 1 et la structure de liaison 4.

Dans le mode de réalisation préféré, représenté par les figures 2 à 5, cet axe 10 est orienté, quand le dispositif de maintien est assemblé aux os maxillaires, selon une direction sensiblement normale au plan sagittal prédéfini, c'est-à-dire qu'il forme un angle inférieur à 10° avec la normale à ce plan sagittal.

### c. Blocage de la liaison pivot

De façon avantageuse, l'élément de liaison central 11 est équipé d'un levier 111 permettant d'entrainer en rotation une pièce excentrique qui est en contact avec la tige cylindrique centrale 41, dans l'ouverture 110 de l'élément de liaison central 11. Ce levier 111 peut être actionné pour modifier la position de la pièce excentrique, entre une position verrouillée dans laquelle elle exerce une pression sur la tige 41, afin de bloquer sa position par rapport à l'élément de liaison central 11, et une position ouverte dans laquelle elle n'exerce pas de pression suffisante sur la tige 41 pour l'empêcher de pivoter ou de coulisser dans l'élément de liaison central 11.

### 3. Éléments d'ancrage latéraux

Les éléments d'ancrage latéraux 2 et 3 sont avantageusement symétriques, et sont destinés à être utilisés de façon symétrique par rapport à l'axe sagittal précédemment défini. En conséquence, seul l'élément d'ancrage latéral 2 est décrit en détail ci-après. Cet élément d'ancrage latéral 2 est destiné à être fixé à la portion supérieure droite 52 de l'os maxillaire. Il comporte un élément de liaison 21 qui est destiné à faire partie d'une liaison pivot-glissant, d'axe 20, entre l'élément d'ancrage latéral 2 et la structure de liaison 4, et une patte de fixation 22.

### a. Patte de fixation

La patte de fixation 22 est destinée à permettre la fixation de l'élément d'ancrage latéral 2 sur la portion supérieure droite 52 de l'os maxillaire du patient, à côté du nez de celui-ci. Cette patte de fixation 22 présente une surface apte à être mise en contact avec l'os du patient, et un trou de passage de vis. Ce trou permet le passage d'une vis de fixation qui peut être vissée par le chirurgien dans l'os maxillaire du patient, pour fixer l'élément d'ancrage latéral 2.

### b. Elément de liaison 21

L'élément de liaison 21 présente une ouverture 210 dans laquelle est introduite une tige cylindrique latérale 42 de la structure de liaison 4, afin que cet élément de liaison 21 et la tige cylindrique latérale 42 forment ensemble une liaison pivot-glissant, d'axe 20, entre l'élément d'ancrage latéral 2 et la structure de liaison 4.

Dans le mode de réalisation préféré, représenté par les figures 2 à 5, cet axe 20 est orienté, quand le dispositif de maintien est assemblé aux os maxillaires, selon une direction sensiblement parallèle au plan sagittal prédéfini. Ainsi, cet axe 20 s'étend dans une direction sensiblement orthogonale à la direction de l'axe 10.

### c. Blocage de la liaison pivot

De façon avantageuse, l'élément de liaison 21 est équipé d'un levier 211, semblable au levier 111 décrit précédemment, lié à une pièce excentrique en contact avec la tige cylindrique latérale 42, le levier entrainant la pièce excentrique entre une position verrouillée dans laquelle elle exerce une pression sur la tige 42, afin de bloquer sa position par rapport à l'élément de liaison 21, et une position ouverte dans laquelle elle n'exerce pas de pression suffisante sur la tige 42 pour l'empêcher de pivoter ou de coulisser dans l'élément de liaison 21.

### d. Elément d'ancrage gauche

L'élément d'ancrage latéral 3, destiné à être fixé à la portion supérieure gauche 53 de l'os maxillaire, est avantageusement symétrique à l'élément d'ancrage latéral 2. Il présente ainsi une patte de fixation 32 un élément de liaison 31, présentant une ouverture 310 qui permet de former une liaison pivot-glissant, d'axe 30, avec une tige latérale 43 la structure de liaison 4. Ces éléments sont avantageusement identiques, ou quasiment identiques, à ceux de l'élément d'ancrage 2.

### 4. Structure de liaison

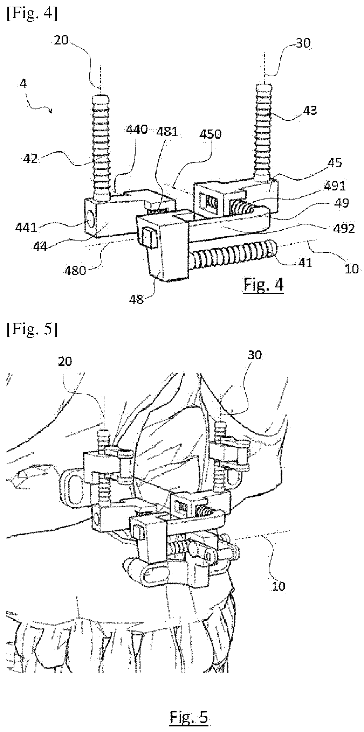

La structure de liaison 4 est représentée par la [Fig.4]. Comme expliqué précédemment, elle présente dans le mode de réalisation représenté une tige centrale cylindrique 41 s'étendant le long d'un premier axe 10, et deux tiges cylindriques latérales 42 et 43 s'étendant respectivement le long d'axes 20 et 30. De façon préférentielle, la structure de liaison est configurée pour que chacun de ces axes 20 et 30 puisse être sensiblement orthogonal à l'axe 10.

Ces trois tiges cylindriques 41, 42 et 43 forment chacune une liaison pivot-glissant verrouillable avec l'un des éléments d'ancrage 1, 2 et 3, de telle sorte que cette structure de liaison 4 relie les différents éléments d'ancrage 1, 2 et 3, quand ceux-ci sont fixés aux os maxillaires du patient. Quand la structure de liaison relie les éléments d'ancrage 1, 2 et 3 fixés sur les os maxillaires du patient, il est possible de déplacer de façon contrôlée l'élément d'ancrage central 1 par rapport aux éléments d'ancrage latéraux 2 et 3, en agissant sur les liaisons pivot-glissant verrouillables entre les points d'ancrage et la structure de liaison.

Dans le mode de réalisation représenté, cette structure de liaison 4 est avantageusement composée de plusieurs pièces mobiles les unes par rapport aux autres, afin de multiplier les possibilités de déplacement contrôlés des points d'ancrages les uns par rapport aux autres. Ainsi, dans ce mode de réalisation, la structure de liaison 4 est composée de quatre pièces distinctes : deux supports de tiges latérales 44 et 45, un support de tige centrale 48, et une équerre de liaison 49.

### a. Supports de tiges latérales 44 et 45

Les supports de tiges latérales 44 et 45 portent respectivement les tiges latérales cylindriques 42 et 43, qui ont été décrites précédemment. Ces supports de tiges latérales sont avantageusement identiques, ou quasi-identiques.

Le support de tige latérale 44 est fixé à l'une des extrémités de la tige latérale 42, et permet la jonction de cette tige 42 avec les autres pièces de la structure de liaison 4. Ce support de tige latérale 44 présente une ouverture destinée à recevoir une tige cylindrique pour former, avec cette tige, une liaison pivot-glissant, d'axe 440, entre cette tige et le support de tige latérale 44. Dans le mode de réalisation préféré, représenté par les figures 2 à 5, cet axe 440 est orienté selon une direction orthogonale à la direction de l'axe 20, qui correspond à la direction longitudinale de la tige cylindrique latérale 42.

De façon avantageuse, une vis peut être introduite dans un trou 441 prévu dans le support de tige latérale 44, afin de permettre le blocage de la position de la tige introduite dans l'ouverture du support de tige latérale 44. Il est à noter que, dans d'autres mode de réalisation de l'invention, il est également possible que cette vis soit remplacée par d'autres moyens de blocage, comme par exemple par un levier lié à une pièce excentrique en contact avec la tige cylindrique à bloquer..

De la même façon, le support de tige latérale 45 présente une ouverture destinée à recevoir une tige cylindrique pour former, avec cette tige, une liaison pivot-glissant entre cette tige et le support de tige latérale 43. Cette liaison pivot-glissant, qui peut être bloquée par une vis ou par tout autre moyen de blocage, est selon un axe 450 orthogonal à l'axe 30, qui correspond à la direction longitudinale de la tige cylindrique latérale 43.

### b. Support de tige centrale 48

Le support de tige centrale 48 est fixé à l'une des extrémités de la tige centrale 41, et permet la jonction de cette tige centrale 41 avec les autres pièces de la structure de liaison 4.

Ce support de tige centrale 48 porte une tige cylindrique de liaison 481 qui possède une forme allongée, cylindrique et complémentaire à la structure interne de l'ouverture du support de la tige latérale 44. Cette tige cylindrique de liaison 481 est orientée selon une direction orthogonale à la direction de la tige latérale 42.

Cette tige cylindrique de liaison 481 est introduite dans l'ouverture du support de tige latérale 44 pour former la liaison pivot-glissant d'axe 440 entre le support de tige centrale 48 et le support de tige latérale 44.

### c. Liaison glissière avec l'équerre de liaison 49

Le support de tige centrale 48 présente également une ouverture de section carrée destinée à recevoir une tige 492 de section carrée pour former, avec cette tige, une liaison glissière, d'axe 480, entre cette tige de section carrée 492 et le support de tige centrale 41. Dans le mode de réalisation préféré, représenté par les figures 2 à 5, cet axe 480 est parallèle à la direction de l'axe 10.

La tige de section carrée 492 fait partie de l'équerre 49, qui est destinée à relier le support de tige centrale 48 et le support de tige latérale 45. La liaison glissière entre cette équerre 49 et le support de tige centrale 48 permet avantageusement de régler la largeur du dispositif de positionnement, et plus particulièrement l'écartement entre les deux éléments d'ancrage latéraux 2 et 3, en fonction de la morphologie du patient. Cette liaison glissière peut avantageusement être verrouillée par tout moyen de blocage réversible connu de l'homme du métier, comme par exemple par une vis ou par une pièce excentrique entrainée en rotation par un levier.

### d. Equerre de liaison 49

L'équerre de liaison 49 est constituée de deux tiges s'étendant perpendiculairement l'une à l'autre : la tige 492 de section carrée, décrite précédemment, qui est orientée selon l'axe 480, et une tige cylindrique de liaison 491 orientée selon un axe 450 perpendiculaire à cet axe 480.

La tige cylindrique de liaison 491 de l'équerre de liaison 49 possède une forme allongée, cylindrique et complémentaire à la structure interne de l'ouverture du support de la tige latérale 45. Avantageusement, la forme du support de tige centrale 48 et de l'équerre de liaison 49, ainsi que la conformation de la liaison glissière entre ces pièces, assure que la tige cylindrique de liaison 491 et la tige cylindrique de liaison 481 sont orientées parallèlement l'une à l'autre et dans des directions orthogonales à la direction de l'axe 10.

Cette tige cylindrique de liaison 491 est introduite dans l'ouverture du support de tige latérale 43 pour former la liaison pivot-glissant d'axe 450 entre l'équerre de liaison 49 et le support de tige latérale 43. Cet axe 450 est orienté selon une direction parallèle à l'axe 440 précédemment décrit.

### 5. Tiges rainurées

Selon un mode de réalisation particulièrement avantageux, au moins certaines des tiges cylindriques mises en œuvres pour former des liaisons pivot-glissants, et de préférence toutes ces tiges cylindriques, présentent des rainures périphériques réparties sur leur longueur, par exemple à raison d'une rainure tous les millimètres.

Ainsi, dans le mode de réalisation représenté par les figures 2 à 5, la tige centrale cylindrique 41, les tiges latérales cylindriques 42 et 43 et les tiges cylindriques de liaison 481 et 491 présentent de telles rainures périphériques.

Ces rainures sont destinées à coopérer avec un curseur placé dans l'ouverture dans laquelle la tige est introduite pour former une liaison pivot-glissant. De façon avantageuse, cette coopération entre le curseur et une rainure de la tige permet de maintenir la position en translation de la tige dans l'ouverture, tant qu'une force suffisante n'est pas appliquée. Les coulissements accidentels ou incontrôlés de la tige sont ainsi évités, par exemple quand on cherche à faire pivoter la tige sans la laisser coulisser.

Par ailleurs, le curseur est avantageusement conformé pour que le coulissement de la tige dans l'ouverture se fasse en « pas à pas », avec un retour sensoriel, comprenant notamment un son de type « clic », à chaque fois que le curseur passe d'une rainure à l'autre. Il est ainsi possible de connaitre avec précision l'amplitude d'une translation. Enfin, les rainures fournissent un repère visuel aidant également à connaitre précisément l'amplitude de cette translation.

### 6. Utilisation du dispositif de positionnement

L'utilisation d'un dispositif de positionnement des os maxillaires selon l'invention, lors d'une opération chirurgicale d'« ostéotomie de Lefort 1 », nécessite l'exécution de différentes étapes opératoires par le chirurgien.

### a. Première mise en place du dispositif de positionnement

Lors d'une telle opération chirurgicale, le chirurgien doit réaliser une incision de la muqueuse buccale, au niveau de la lèvre supérieure du patient. Cette incision permet l'accès aux os maxillaires, lesquels doivent être clairement exposés.

Le chirurgien peut alors positionner le dispositif de positionnement selon l'invention contre les os maxillaires du patient, et réaliser des trous aux endroits de passage des vis des pattes de fixation 12 et 13 situés sur la portion inférieure 51 des os maxillaires et des pattes de fixation 22 et 32 situés respectivement sur les portions supérieures droite 52 et gauche 53 des os maxillaires. Après cette première implantation du dispositif de positionnement selon l'invention, le chirurgien peut retirer ce dispositif, afin de libérer son accès aux os maxillaires. Le dispositif de positionnement est alors conservé avec les mêmes réglages que lors de cette première implantation.

### b. Découpe osseuse

Le chirurgien réalise ensuite la découpe osseuse 50 constituant l'« ostéotomie de Lefort 1 », afin de libérer la portion inférieure 51 des os maxillaires portant les dents des portions supérieures droite 52 et gauche 53. Cette découpe 50 de l'os est visible sur les figures 1, 3 et 5.

### c. Déplacement de la partie inférieure des os maxillaires

Le chirurgien peut alors fixer le dispositif de positionnement selon l'invention sur les portions supérieures 52 et 53 des os maxillaires et sur la portion inférieure 51 des os maxillaires, en vissant des vis au niveau des pattes de fixation 12, 13, 22 et 32, au niveau des trous qu'il a réalisés précédemment. Pour cette fixation, le dispositif de positionnement conserve les mêmes réglages que lors de la première implantation. Ainsi, le dispositif de positionnement est fixé dans une position dite initiale, dans laquelle il maintient la portion inférieure 51 des os maxillaires dans la même position, par rapport aux portions supérieures 52 et 53 des os maxillaires, qu'avant la découpe osseuse.

Une fois que le dispositif de positionnement est fixé dans cette position initiale, le chirurgien peut ajuster les réglages du dispositif de positionnement, afin de changer la position de la portion inférieure 51 des os maxillaires, qui est mobile, par rapport aux portions supérieures 52 et 53 des os maxillaires fixes. Pour ce faire, le chirurgien peut régler cette position avec précision par divers mouvements qui sont rendus possibles par les liaisons pivot-glissant du dispositif de positionnement.

### d. Déplacements antéro-postérieurs et rotation

Le chirurgien peut notamment effectuer des déplacements antéro-postérieurs de la portion inférieure 51 des os maxillaires par rapport à la portion supérieure droite 52, dans un plan théorique transversal sensiblement normal au plan sagittal. Le chirurgien peut réaliser ces déplacements antéro-postérieurs par une translation pas-à-pas, selon les axes 440 et 450, des tiges cylindriques de liaison 481 et 491 par rapport, respectivement, aux supports de tige latérale 44 et 45.

Une fois la position souhaitée obtenue, le chirurgien peut alors fixer cette position en verrouillant, par le serrage de vis, la position des tiges cylindriques de liaison 481 et 491 par rapport, respectivement, aux supports de tige latérale 44 et 45.

Le chirurgien peut réaliser une rotation de la portion inférieure 51 des os maxillaires par rapport à la portion supérieure gauche 53, en réalisant des translations d'amplitudes différentes de la tige cylindriques de liaison 481 par rapport au support de tige latérale 44 et de la tige cylindrique de liaison 491 par rapport au support de tige latérale 45. Dans un tel cas, les supports de tige latérale 44 et 45 pourront pivoter légèrement, respectivement autour des axes 20 et 30, et la tige de section carrée 492 de l'équerre 49 pourra coulisser légèrement dans le support de tige centrale 48, afin que les tiges cylindriques de liaison 481 et 491 restent parallèles l'une à l'autre. Pour permettre ces mouvements, les moyens de blocage associés à ces pivots glissants et glissières devront être préalablement débloqués.

### e. Déplacements verticaux et rotation

Le chirurgien a la possibilité de réaliser des déplacements verticaux de la portion inférieure 51 par rapport aux portions supérieures 52 et 53 des os maxillaires dans un plan frontal, avec un contrôle de la distance canthus/canine gauche et droite. Le chirurgien peut effectuer ces déplacements verticaux par une translation pas-à-pas, selon les axes 20 et 30, des tiges latérales cylindriques 42 et 43 par rapport, respectivement, aux éléments d'ancrage latéraux 2 et 3.

Une fois la position souhaitée obtenue, le chirurgien peut alors fixer cette position en verrouillant, par le serrage des leviers 211 et 311, la position des tiges latérales cylindriques 42 et 43 par rapport, respectivement, aux éléments d'ancrage latéraux 2 et 3.

Le chirurgien peut également réaliser une rotation de la portion inférieure 51 des os maxillaires par rapport à la portion supérieure gauche 53, en réalisant des translations d'amplitude différente de la tige latérale cylindrique 42 par rapport à l'élément d'ancrage latéral 2 et de la tige latérale cylindrique 43 par rapport à l'élément d'ancrage latéral 3. Ce mouvement différent pourra entrainer des mouvements de faible amplitude au niveau des autres articulations de la structure de liaison 4, notamment des rotations de la portion inférieure 51 autour des axes 440 ou 450. Pour permettre ces mouvements, les moyens de blocage associés à ces pivots glissants devront être préalablement débloqués.

### f. Déplacements gauche/droite et de rotation

Le chirurgien a la possibilité de réaliser des déplacements vers la gauche ou vers la droite de la portion inférieure 51 par rapport aux portions supérieures 52 et 53 des os maxillaires dans un plan transverse perpendiculaire au plan sagittal prédéfini. Le chirurgien peut réaliser ces mouvements vers la gauche ou vers la droite par une translation pas-à-pas, selon l'axe 10, de la tige centrale cylindrique 41 par rapport à l'éléments d'ancrage central 1.

Le chirurgien a également la possibilité de réaliser des mouvements de rotation de la portion inférieure 51 autour de cet axe 10, par rapport aux portions supérieures 52 et 53 des os maxillaires. Cette rotation peut être obtenue par le pivotement de la tige centrale cylindrique 41 par rapport à l'éléments d'ancrage central 1.

Une fois la position souhaitée obtenue, le chirurgien peut alors bloquer la liaison pivot-glissant en actionnant le levier 111.

### g. Retrait du dispositif

Ces divers alignements permettent d'obtenir un bon alignement de l'arcade dentaire, portée par la portion inférieure 51 des os maxillaires, avec la ligne du regard, c'est-à-dire selon une direction sensiblement normale au plan sagittal prédéfini. Ces alignements permettent d'assurer un bon recentrage du milieu inter-incisif avec le plan sagittal médian. Le dispositif permet également de réaliser des rotations angulaires des maxillaires inférieures autours de l'axe sagittal médian.

L'utilisation du dispositif de positionnement selon l'invention, qui peut avantageusement être utilisé de façon standardisée pour une grande variété de patients, permet donc au chirurgien d'effectuer un grand nombre de mouvements différents de la portion inférieure des os maxillaires du patient, par rapport aux portions supérieures de ces os maxillaires. Ces mouvements peuvent avantageusement être adaptés au cours de l'opération, si les circonstances imposent au chirurgien d'adapter le protocole opératoire préétabli.

Une fois la position souhaitée obtenue, le chirurgien réalise la fixation finale de la portion inférieure 51 avec les portions supérieures droite 52 et gauche 53 des os maxillaires par des plaques et vis en titane, en commençant de préférence par les plaques les plus postérieures, au niveau du cintre zygomatique.

Le chirurgien peut alors retirer le dispositif de positionnement en dévissant les vis situées au niveau des pattes de fixation 12, 13, 22 et 23. Une fois le dispositif de positionnement retiré, il peut finaliser la fixation de la portion inférieure 51 avec les portions supérieures droite 52 et gauche 53 des os maxillaires au niveau de l'orifice piriforme.

## Revendications

1. Dispositif de positionnement des os maxillaires dans le cadre d'une opération chirurgicale d'ostéotomie des os maxillaires d'un patient,
ledit dispositif de positionnement comprend :
- un élément d'ancrage central (1), apte à être fixé aux os maxillaires dudit patient, entre son nez et son arcade dentaire,
- deux éléments d'ancrage latéraux (2, 3), aptes à être fixés aux os maxillaires dudit patient, chacun de l'un des côtés du nez dudit patient,
- une structure de liaison (4) connectée auxdits éléments d'ancrage latéraux (2, 3) et central (1),
**caractérisé en ce que** ladite structure de liaison (4) est articulée pour former trois liaisons pivot-glissant entre ledit élément d'ancrage central (1) et chacun desdits éléments d'ancrage latéraux (2, 3) comprend:
- une première liaison pivot-glissant, orientée selon un premier axe (10),
- une deuxième liaison pivot-glissant, orientée selon un deuxième axe (440, 450) sensiblement orthogonal audit premier axe (10), et
- une troisième liaison pivot-glissant, orientée selon un troisième axe (20, 30) sensiblement orthogonal audit deuxième axe.

2. Dispositif de positionnement selon la revendication 1, **caractérisé en ce que** ladite structure de liaison comprend plusieurs pièces mobiles les unes par rapport aux autres, parmi lesquelles :
- une première pièce connectée audit élément d'ancrage central (1) par ladite première liaison pivot-glissant, et
- une deuxième pièce connectée à ladite première pièce par une desdites deuxième liaison pivot-glissant, et connectée à un premier desdits éléments d'ancrage latéraux (2), par une desdites troisième liaison pivot-glissant.

3. Dispositif de positionnement selon la revendication 2, **caractérisé en ce que** ladite structure de liaison comprend :
- une troisième pièce connectée à ladite première pièce par une liaison glissière orientée selon un axe (480) parallèle audit premier axe (10), et
- une quatrième pièce connectée à ladite troisième pièce par une desdites deuxième liaison pivot-glissant, et connectée au second desdits éléments d'ancrage latéraux (2), par une desdites troisième liaison pivot-glissant.

4. Dispositif de positionnement selon l'une quelconque des revendications précédentes, **caractérisé en ce qu'**au moins une desdites liaisons pivot-glissant est constituée par une ouverture (110, 210, 310) ménagée dans une pièce, dans laquelle est introduite une tige cylindrique (41, 42, 43, 481, 491), ladite tige présentant une pluralité de rainures périphériques réparties sur au moins une partie de sa longueur.

5. Dispositif de positionnement selon la revendication précédente, **caractérisé en ce que** ladite ouverture est conformée pour coopérer avec lesdites rainures pour générer un retour sensitif et/ou sonore à chaque déplacement en translation, d'un pas correspondant à l'écart entre deux rainures successives, de ladite tige dans ladite ouverture.

6. Dispositif de positionnement selon la revendication précédente, **caractérisé en ce que** ladite liaison pivot-glissant est équipée d'un curseur placé dans ladite ouverture (110, 210, 310), ledit curseur étant apte à coopérer avec une rainure de ladite tige (41, 42, 43, 481, 491) pour maintenir la position en translation de ladite tige dans ladite ouverture, tant qu'une force prédéterminée n'est pas appliquée.

7. Dispositif de positionnement selon la revendication précédente, **caractérisé en ce que** ledit curseur est conformé pour générer un son lors de son passage d'une desdites rainures à une autre desdites rainures de ladite tige (41, 42, 43, 481, 491).

8. Dispositif de positionnement selon l'une quelconque des revendications précédentes, **caractérisé en ce qu'**il est équipé de moyens de blocage réversibles d'au moins une desdites liaisons pivot-glissant.

9. Dispositif de positionnement selon la revendication précédente, **caractérisé en ce qu'**au moins un desdits moyens de blocage réversible comprend une came excentrique associée à un levier (111, 211, 311).

## Patentansprüche

1. Vorrichtung zur Positionierung der Kieferknochen durch einen chirurgischen Osteotomie-Eingriff in den Kieferknochen eines Patienten,
wobei die Vorrichtung zur Positionierung
- ein Zentralankerelement (1), das an den Kieferknochen des Patienten zwischen Nase und Zahnbogen befestigbar ist;
- zwei Seitenankerelemente (2, 3), die an den Kieferknochen des Patienten jeweils auf einer Seite der Nase des Patienten befestigbar sind;
- eine Verbindungsstruktur (4), die mit den Seitenankerelementen (2, 3) und dem Zentralankerelement (1) verbunden ist, umfasst;
**dadurch gekennzeichnet, dass** die Verbindungsstruktur (4) zu drei Schwenk- und Gleitverbindungen zwischen dem Zentralankerelement (1) und jedem der Seitenankerelemente (2, 3) gegliedert ist und umfasst
- eine erste Schwenk- und Gleitverbindung, die nach einer ersten Achse (10) ausgerichtet ist,
- eine zweite Schwenk- und Gleitverbindung, die nach einer zweiten Achse (440, 450) im Wesentlichen orthogonal auf die erste Achse (10) ausgerichtet ist und
- eine dritte Schwenk- und Gleitverbindung, die nach einer dritten Achse (20, 30) im Wesentlichen orthogonal auf die zweite Achse ausgerichtet ist.

2. Vorrichtung zur Positionierung nach Anspruch 1 **dadurch gekennzeichnet, dass** die Verbindungsstruktur mehrere miteinander bewegliche Teile umfasst, wobei
- ein erstes Teil mit dem Zentralankerelement (1) durch die erste Schwenk- und Gleitverbindung verbunden ist und
- ein zweites Teil durch eine der zweiten Schwenk- und Gleitverbindungen mit dem ersten Teil verbunden ist und durch eine der dritten Schwenk- und Gleitverbindungen mit einem der ersten Seitenankerelemente (2) verbunden ist.

3. Vorrichtung zur Positionierung nach Anspruch 2 **dadurch gekennzeichnet, dass** die Verbindungsstruktur
- ein drittes Teil, das durch eine Gleitverbindung, die nach einer Achse (480) parallel zur ersten Achse (10) ausgerichtet ist, mit dem ersten Teil verbunden ist und
- ein viertes Teil, das durch eine der zweiten Schwenk- und Gleitverbindungen mit dem dritten Teil verbunden ist und durch eine der dritten Schwenk- und Gleitverbindungen mit den zweiten der Seitenankerelemente (2) verbunden ist, umfasst.

4. Vorrichtung zur Positionierung nach einem der vorhergehenden Ansprüche **dadurch gekennzeichnet, dass** zumindest eine der Schwenk- und Gleitverbindungen aus einer Öffnung (110, 210, 310) in einem Raum ausgebildet ist, in die ein zylindrischer Schaft (41, 42, 43, 481, 491) eingeführt wird, wobei der Schaft eine Mehrzahl von umlaufenden Nuten, die sich über zumindest einen Teil ihre Länge verteilen, aufweist.

5. Vorrichtung zur Positionierung nach dem vorhergehenden Anspruch **dadurch gekennzeichnet, dass** die Öffnung so ausgebildet ist, dass sie mit den Nuten zusammenwirkt, um bei jeder Translationsverschiebung des Schafts in der Öffnung um einen Teilungsabschnitt, der dem Abstand zwischen zwei aufeinanderfolgenden Nuten entspricht, eine sensorische und/oder akustische Rückmeldung zu erzeugen.

6. Vorrichtung zur Positionierung nach dem vorhergehenden Anspruch **dadurch gekennzeichnet, dass** die Schwenk- und Gleitverbindung mit einem in der Öffnung (110, 210, 310) angebrachten Schieber ausgestattet ist, wobei der Schieber mit einer Nut des Schafts (41, 42, 43, 481, 491) zusammenwirken kann, um die Translationsposition des Schafts in die Öffnung, solange keine vorgegebene Kraft angewendet wird, zu sichern.

7. Vorrichtung zur Positionierung nach dem vorhergehenden Anspruch **dadurch gekennzeichnet, dass** der Schieber so ausgebildet ist, dass er bei seinem Übergang von einer der Nuten zu einer anderen der Nuten des Schafts (41, 42, 43, 481, 491) einen Ton erzeugt.

8. Vorrichtung zur Positionierung nach einem der vorhergehenden Ansprüche **dadurch gekennzeichnet, dass** sie mit umkehrbaren Sperrmitteln, der zumindest einer der Schwenk- und Gleitverbindungen ausgestattet ist.

9. Vorrichtung zur Positionierung nach dem vorhergehenden Anspruch **dadurch gekennzeichnet, dass** zumindest ein der umkehrbaren Sperrmittel eine exzentrische Nocke (111, 211, 311) mit einem Hebel umfasst.

## Claims

1. A device for positioning the maxillary bones during an osteotomy surgical operation on a patient's maxillary bones,
the positioning device comprising:
- a central anchoring element (1) suitable for being fastened to the patient's maxillary bones between their nose and dental arch,
- two lateral anchoring elements (2, 3) suitable for being fastened, each on one side of the patient's nose, to the patient's maxillary bones, and
- a linking structure (4) connected to the lateral anchoring elements (2, 3) and the central anchoring element (1),
**characterised in that** the linking structure (4) is articulated so as to form three sliding pivot links between the central anchoring element (1) and each of the lateral anchoring elements (2, 3), the linking structure (4) comprising:
- a first sliding pivot link oriented along a first axis (10),
- a second sliding pivot link oriented along a second axis (440, 450) which is substantially orthogonal to the first axis (10), and
- a third sliding pivot link, oriented along a third axis (20, 30) which is substantially orthogonal to the second axis.

2. A positioning device according to claim 1, **characterised in that** the linking structure comprises a plurality of parts which are movable relative to one another, including:
- a first part connected to the central anchoring element (1) by the first sliding pivot link, and
- a second part connected to the first part by one of the second sliding pivot links and to a first of the lateral anchoring elements (2) by one of the third sliding pivot links.

3. A positioning device according to claim 2, **characterised in that** the linking structure comprises:
- a third part connected to the first part by a sliding link oriented along an axis (480) which is parallel to the first axis (10), and
- a fourth part connected to the third part by one of the second sliding pivot links and to the second of the lateral anchoring elements (2) by one of the third sliding pivot links.

4. A positioning device according to any one of the preceding claims, **characterised in that** at least one of the sliding pivot links is formed by an opening (110, 210, 310) made in a part, in which a cylindrical rod (41, 42, 43, 481, 491) is received, the rod having a plurality of peripheral grooves distributed over at least a portion of its length.

5. A positioning device according to the preceding claim, **characterised in that** the opening is shaped to cooperate with the grooves to generate tactile and/or auditory feedback for each translational movement of the rod in the opening by a step corresponding to the spacing between two successive grooves.

6. A positioning device according to the preceding claim, **characterised in that** the sliding pivot link is provided with a slider which is arranged in the opening (110, 210, 310) and is suitable for cooperating with a groove of the rod (41, 42, 43, 481, 491) to maintain the translational position of the rod in the opening as long as a predetermined force is not applied.

7. A positioning device according to the preceding claim, **characterised in that** the slider is shaped to generate a sound when it passes from one of the grooves to another of the grooves of the rod (41, 42, 43, 481, 491).

8. A positioning device according to any one of the preceding claims, **characterised in that** it is provided with reversible locking means for at least one of the sliding pivot links.

9. A positioning device according to the preceding claim, **characterised in that** at least one of the reversible locking means comprises an eccentric cam associated with a lever (111, 211, 311).
